# EUROPEAN PATENT APPLICATION

(11) **EP 1 179 328 A1**
(43) Date of publication of application: **13.02.2002**
(21) Application number: 00124469.8
(22) Date of filing: 08.11.2000
(51) Int. Cl.: A61F 7/00

(54) **Device for preventing body hypo/hyperthermia incorporating an open-cell dispersion system**

(30) Priority: 08.08.2000 US 634531
(71) Applicant: GAYMAR INDUSTRIES INC., Orchard Park, New York 14127 (US)
(72) Inventor: Buchanan, Richard W., Hamburg, New York 14075 (US); Martin, Keith, Orchard Park, New York 14127 (US); Stewart, Thomas P., Orchard Park, New York 14127 (US)
(74) Representative: Koepe, Gerd L., Dipl.-Chem.

(57) **Abstract**

The present invention is directed to a hypo/hyperthermia system. The system has a temperature altering unit (10), a first conduit (16) and an open-cell dispersing unit (12). The temperature altering unit receives a medium at ambient, heated, or cooled temperatures, and converts the medium into a second medium at a desired temperature. The first conduit transfers the second medium to the open-cell dispersing unit. And the open-cell dispersing unit has a receiving aperture (14), a central chamber and an open-cell instrument. The receiving aperture receives the first conduit. The central chamber receives the second medium. While the open-cell instrument surrounds the central chamber, except where the receiving aperture is located, so the second medium is distributed throughout the open-cell instrument to direct the second medium in all directions in a predetermined area.

## Description

### FIELD OF INVENTION

The present invention relates to a unit that disperses thermal energy.

### BACKGROUND OF THE PRESENT INVENTION

Gaymar Industries, Inc., Augustine Medical, Inc., and Mallinckrodt Inc. manufacture heating/cooling unit devices, e.g., the THERMACARE® unit, the BAIR HUGGER® unit, the WARM TOUCH® unit respectively, that draw ambient air into the device and expel the air into a hose at a predetermined temperature ("second air"). The hose of each heating unit has two ends, a receiving end which receives the second air and a distributing end which distributes the second air to a hypo/hyperthermia blanket.

The hypo/hyperthermia blanket, normally disposable, has a receiving aperture, an air circulation design within the blanket, and a plurality of outlet apertures. The receiving aperture receives the distributing end of the hose and the second air into the blanket. Once the second air is within the blanket, the air circulation design ensures the second air goes throughout the interior of the blanket. The second air then escapes from the blanket to a patient through the plurality of outlet apertures. This system is extremely well recognized and known to those skilled in the art of hypo/hyperthermia.

A problem with the blanket is that the unit is not always practical for all applications in surgery or other uses. As such, many entities have modified the way they use the heating unit devices. For example, it has been found at certain hospitals that they wrap a towel around the distributing end of the hose, and places the towel-wrapped hose near a patient. This use of the heating unit and the hose is not advisable because it is possible that if the second air expels into a concentrated area of the patient because the towel funnels the second air there, then the second air could possibly burn the patient at that particular area. This outcome is not desired and should be avoided. The present invention solves this problem and many others.

### SUMMARY OF THE INVENTION

The present invention is directed to a hypo/hyperthermia system. The system has a temperature altering unit, a first conduit and an open-cell dispersing unit. The temperature altering unit receives a medium at ambient, heated, or cooled temperatures, and converts the medium into a second medium at a desired temperature. The first conduit transfers the second medium to the open-cell dispersing unit. And the open-cell dispersing unit has a receiving aperture, a central chamber and an open-cell instrument. The receiving aperture receives the first conduit. The central chamber receives the second medium. While the open-cell instrument surrounds the central chamber, except where the receiving aperture is located, so the second medium is distributed throughout the open-cell instrument to direct the second medium in all directions in a predetermined area.

### BRIEF DESCRIPTION OF THE DRAWINGS

Figure 1 is a view illustrating the present invention.
Figures 2 - 7 are alternative embodiments of Figure 1.
Figures 8 to 13 are enlarged and alternative embodiments of the low thermally conductive unit.

### DETAILED DESCRIPTION OF THE INVENTION

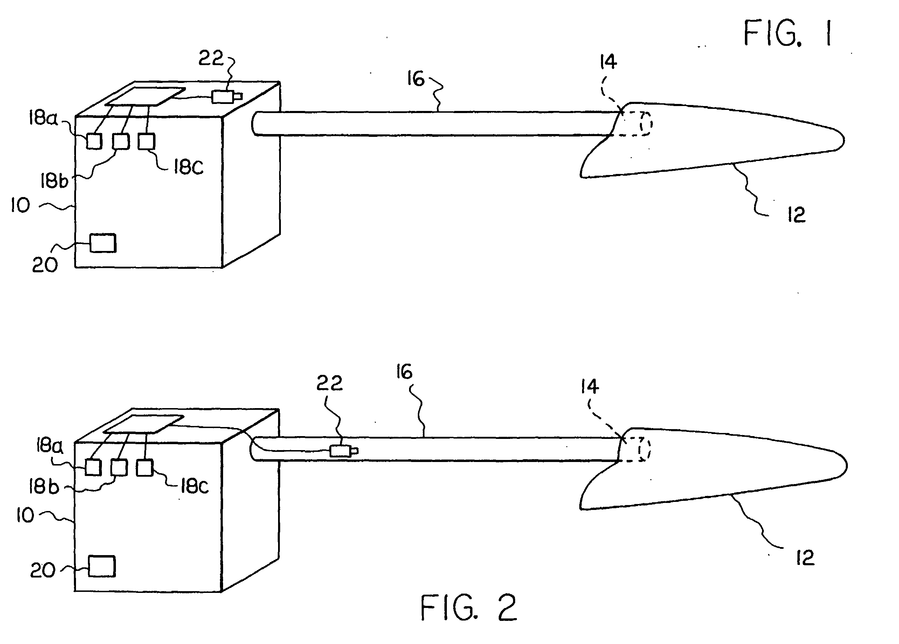

Referring to Figure 1, the present invention details a conventional heating device 10, for example, the THERMACARE® blowers by Gaymar Industries, Inc. of Orchard Park, New York, which are designed to interconnect with a low thermally conductive dispersing unit 12. The low thermally conductive dispersing unit 12 is any conventional open-cell material that disperses the medium, like air, in all possible and/or predetermined directions. The term open-cell material includes foam and foam-like material, cloth and cloth-like material, polymeric material with or without tubes, and metallic material with or without tubes.

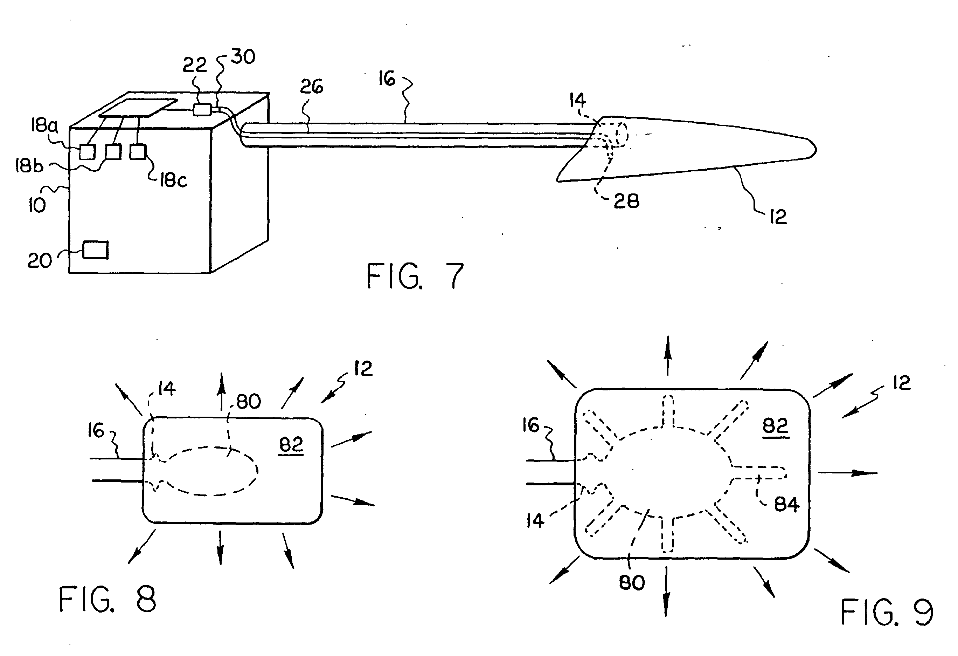

The low thermally conductive dispersing unit 12, for example in the one embodiment illustrated in Figure 8, has an aperture 14 to receive the second air and the distributing end of the hose 16 which is a part of the conventional heating device 10, a central chamber 80 where the second air is directed into from the hose 16, and a plurality of open-cells in a foam and foam-like material, cloth and cloth-like material, polymeric material with or without tubes, and metallic material with or without tubes instrument 82 that surrounds the central chamber 80, except where the aperture 14 is located. The hose 16 and the low thermally conductive dispersing unit 12 are securely attached to each other to prevent the second medium from being directed to one particular point of a predetermined area, like a shoulder, neck, knee, and lower back, and possibly injuring the patient. One means of securely attaching them together is by having the aperture have a male thread system and the distributing end 16A have a corresponding female thread system, or vice versa, or a pressure fit system, or other conventional mating systems known to those skilled in the art.

The second air, as set forth above, is generated in the conventional heating units 10. From there, the second air then traverses through the hose 16 into the central chamber 80 and throughout the instrument 82. From the instrument 82, the second air disperses in every direction. The second air is then dispersed to the patient or the room.

Alternatively, the low thermally conductive disbursing unit 12, as shown in Figure 9, can have a plurality of directional chambers 84 that further direct the second air throughout the instrument 82.

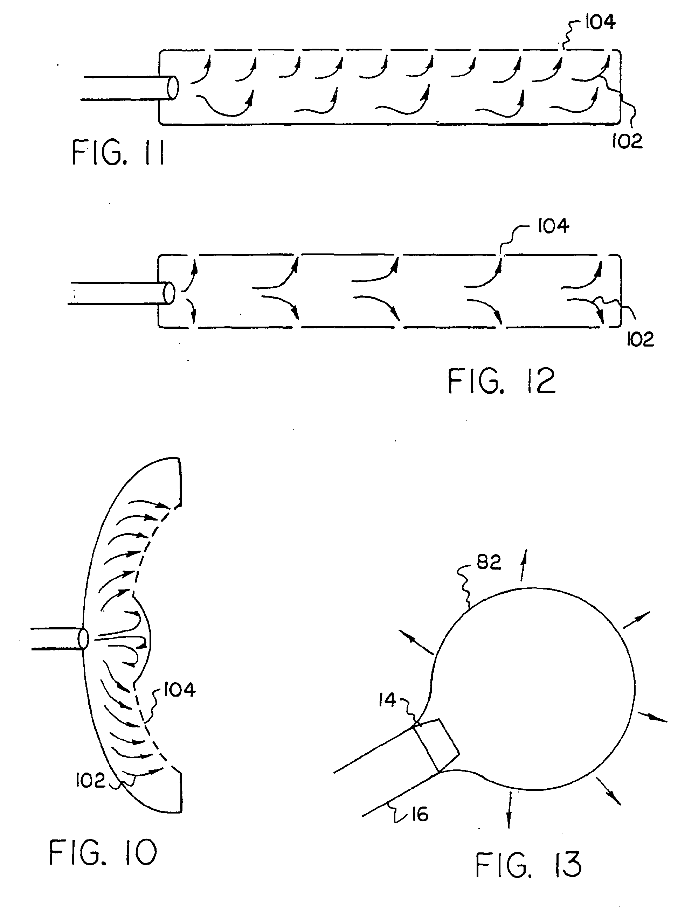

Obviously, the low thermally conductive dispersing unit 12 can be any desired shape. For example, the dispersing unit 12 can be a bedtime carrying object for children like a teddy bear or a tiger, a baseball bat, a beach ball, a hockey puck, a banana as shown in Figure 10 which directs the second air (arrows 102) through predetermined apertures 104, a lumbar/knee support system as shown in Figures 11 and 12 which directs the second air 102 through apertures on either both sides of the system (Figure 12) or one side (Figure 11), or any other imaginable design. An advantage of the low thermally conductive dispersing unit 12 over the conventional blanket for such temperature devices 10, is that the unit 12 is designed to be re-used or, alternatively, be disposable.

In many conventional temperature devices 10, the temperature devices 10 have numerous temperature switches 18 (a,b,c), and at least one on/off switch 20. These switches 18 (a,b,c), 20 are conventional switches. Examples of conventional switches include touch pads and throw-switches, which are conventional light switches.

Turning to the temperature switches 18, temperature device 10 is designed so only one temperature switch 18 can be operable at one time. Accordingly, when a user presses one switch 18a, all other switches 18b, 18c are deactivated. Likewise when switch 18b is activated, the switch 18a is deactivated and switch 18c remains deactivated.

Each temperature switch 18a, 18b, 18c activates the temperature device 10 to heat, cool, and/or convey a medium, like air, having a distinct and desired temperature ("second medium") setting to the dispersing unit 12. Examples of a distinct temperature include, and are not limited to, 50°F, room temperature, 90°F, 100°F, and 110°F.

Obviously, the medium is a material which can be conveyed from the temperature device 10 to the dispersing unit 12, the unit 12 then distributes the second medium to a patient, and the second medium be at the desired temperature setting. Examples of this medium include gasses such as atmospheric air or even water or other fluids.

The hose 16 directs the second medium from the device 10 to the unit 12. Moreover, the hose 16 can be made of any tubing material. Examples of such material include polymeric, metal, wood, or paper. Accordingly, the hose 16 should be flexible, rigid, disposable, and/or non-disposable. In any embodiment, the hose 16 must transport the second medium.

The dispersing unit 12 receives the second medium. Under normal operating proceedings, the second medium within the unit 12, the hose 16, and the device 10, and within a predetermined time period generate an internal pressure that exceeds a predetermined level of internal pressure.

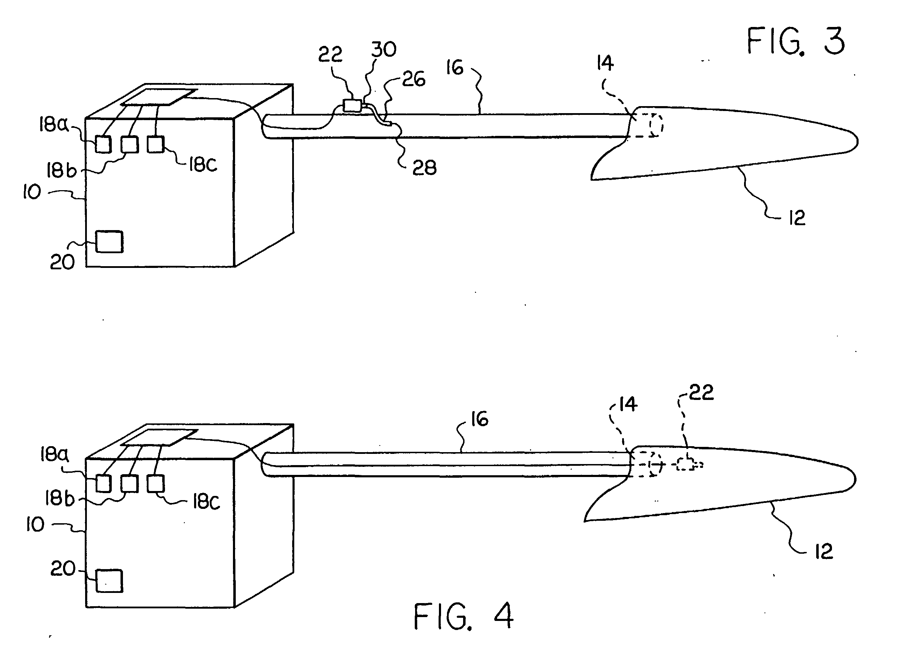

The internal pressure is measured by a pressure sensing apparatus 22. Pressure sensing apparatus 22 is a conventional pressure switch or a pressure transducer. An example of a conventional pressure sensing apparatus is, and not limited to, a Model 500 pressure switch sold by Micro Pneumatic Logic, Inc. of Fort Lauderdale, Florida. The pressure sensing apparatus 22 can be positioned within the device 10 as shown in Figure 1, within the hose 16 as shown in Figure 2, outside the hose 16 as shown in Figure 3, or within the unit 12 as shown in Figure 4. In any position, the pressure sensing apparatus 22 is interconnected with the temperature switches 18.

If the pressure sensing apparatus 22 measures and senses the internal pressure of the second medium, and the internal pressure exceeds the predetermined level, which can be any pressure level, for example, 0.3 inches of H₂O, then any of the temperature switches 18 can be used. If all of the temperature switches 18 can be used, then the user can select the desired temperature setting of the second medium.

In contrast, if the pressure sensing apparatus 22 measures the internal pressure of the medium, and the internal pressure fails to exceed the predetermined level, then the device 10 will generate the second medium at a predetermined temperature setting.

The predetermined temperature setting of the second medium may be one of the temperatures settings designated by switches 18, or an entirely different one (for example, ambient air). In either case, the predetermined temperature is a temperature that will minimize the chances of burning, freezing, and/or damaging the skin or body hair of the patient.

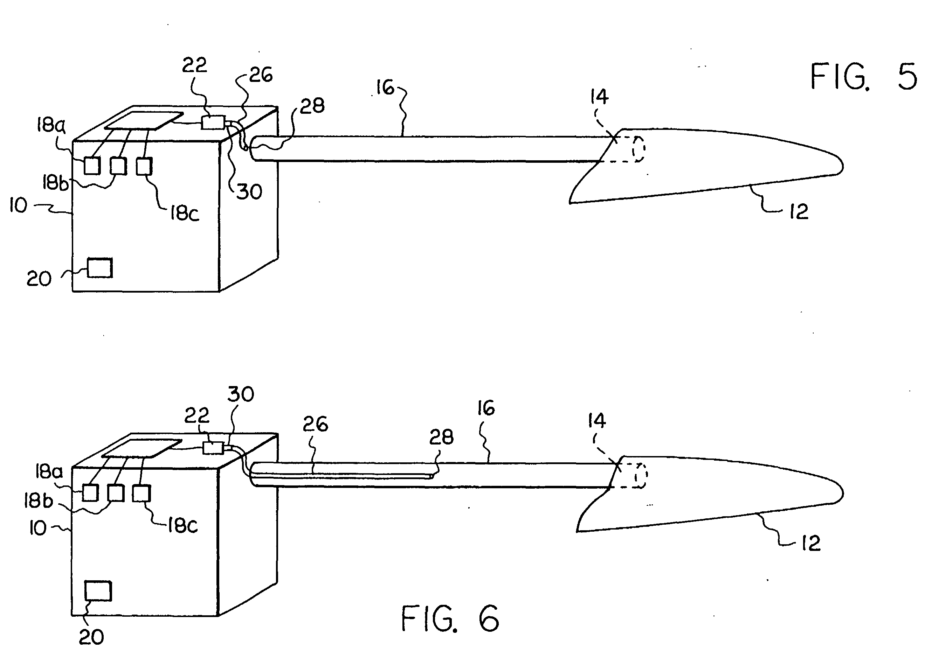

In an alternative embodiment, the pressure sensing apparatus 22 has a second conduit 26. The second conduit 26 has a distal end 28 and a proximal end 30. The proximal end 30 interconnects with the pressure sensing apparatus 22, and the distal end 28 receives the second medium in order to measure the internal pressure within the unit 12 as shown in Figure 5, the first conduit 16 as shown in Figures 3 and 6, or the device 10 as shown in Figure 7. In any embodiment, the second conduit conveys the second medium and inherently the internal pressure to the pressure sensing apparatus 22 so the pressure sensing apparatus 22 measures the internal pressure and thereby controls the switches 18.

Obviously, the alternative embodiment of the device 10 as shown in Figures 1-7 does not have to be used. Instead, conventional heating devices can be used, such as the BAIR HUGGER® unit sold by Augustine Medical of Minnesota, the WARM TOUCH® unit sold by Mallinckrodt Inc. of Missouri, or the THERMACARE® unit which is made and sold by Gaymar Industries, Inc. of Orchard Park, New York.

Alternatively, the low thermally conductive dispersing unit 12, for example in the embodiment illustrated in Figure 13, has an aperture 14 to receive the second air and the distributing end of the hose 16 which is a part of the conventional heating device 10, and a plurality of open-cells in a foam and foam-like material, cloth and cloth-like material, polymeric material with or without tubes, and metallic material with or without tubes instrument 82 that surrounds the aperture 14.

It is intended that the above description of the preferred embodiments of the structure of the present invention and the description of its operation are but one or more enabling best mode embodiments for implementing the invention. Other modifications and variations are likely to be conceived of by those skilled in the art upon a reading of the preferred embodiments and a consideration of the appended claims and drawings. These modifications and variations still fall within the breadth and scope of the disclosure of the present invention.

## Claims

1. A hypo/hyperthermia system comprising:
a temperature altering unit (10) that receives a medium at ambient, heated, or cooled temperatures, and converts the medium into a second medium at a desired temperature;
a first conduit (16) that transfers the second medium to an open-cell dispersing unit (12);
wherein the open-cell dispersing unit (12) has a receiving aperture (14) that receives the first conduit (16), a central chamber (80) that receives the second medium, and an open-cell instrument (82) that surrounds either the central chamber (80), except where the receiving aperture is located, or surrounds the receiving aperture so that the second medium is distributed throughout the open-cell instrument (12) to direct the second medium in predetermined directions.

2. The system of claim 1, wherein the predetermined directions are all directions other than in the aperture direction.

3. The system of claim 1 or claim 2, wherein the medium is air.

4. The system of any of the claims 1 to 3, wherein the open-cell dispersing unit (12) is of a predetermined shape.

5. The system of claim 4, wherein the predetermined shape is selected from a group consisting of a bedtime carrying object for children, a banana, a ball and a planar object.

6. The system of any of the claims 1 to 5, wherein the open-cell dispersing unit (12) has at least one directional chamber (84) extending from the central chamber (80).

7. The system of any of the claims 1 to 6 for use to control the temperature of a patient, preferably the temperature of an animal, more preferably the temperature of a human.

8. The system of any of the claims 1 to 6 for use to control the temperature in a predetermined area, preferably in a predetermined area which is selected from group consisting of a shoulder, a neck, lower back, and knee of a human being.

9. A method of using a hypo/hyperthermia system comprising:
operating a temperature altering unit (10) that receives a medium at ambient, heated, or cooled temperatures, and converts the medium into a second medium at a desired temperature;
connecting a first conduit (16) that transfers the second medium to an open-cell dispersing unit (12);
placing the open-cell dispersing unit (12) that has a receiving aperture (14) that receives the first conduit (16), a central chamber (80) that receives the second medium, and an open-cell instrument (82) that surrounds the central chamber (80), except where the receiving aperture (14) is located, or surrounds the receiving aperture so that the second medium is distributed throughout the open-cell instrument (82) to direct the second medium in predetermined directions onto a predetermined area.

10. The method of claim 12 wherein the predetermined directions are all directions other than in the aperture direction.

11. The method of claim 9 or claim 10, wherein the medium is air.

12. The method of any of the claims 9 to 11, wherein the open-cell dispersing unit (12) is of a predetermined shape.

13. The method of claim 12 wherein the predetermined shape is selected from a group consisting of a bedtime carrying object for children, a banana, a ball and a planar object.

14. The method of any of the claims 9 to 13, wherein the open-cell dispersing unit (12) has at least one directional chamber (84) extending from the central chamber (80).

15. The method of any of the claims 9 to 14, wherein the predetermined area is used to control the temperature of a patient, preferably the temperature of an animal, more preferably the temperature of a human.

16. The method of any of the claims 9 to 15, wherein the predetermined area is selected from group consisting of a shoulder, a neck, lower back, and knee of a human being.
